(19) Europäisches Patentamt
European Patent Office
Office européen des brevets

(11) Numéro de publication: **0 022 117**
**A2**

(12) # DEMANDE DE BREVET EUROPEEN

(21) Numéro de dépôt: 80870032.2

(22) Date de dépôt: 05.06.80

(51) Int. Cl.³: **A 61 K 31/165**
A 61 K 9/18, C 07 C 103/76

(30) Priorité: 08.06.79 BE 195638

(43) Date de publication de la demande: 07.01.81 Bulletin 81/1

(84) Etats Contractants Désignés: CH DE FR GB IT LI NL

(71) Demandeur: Trenker, Ady
478, Avenue Dolez
B-1180 Bruxelles(BE)

(72) Inventeur: Trenker, Ady
478, Avenue Dolez
B-1180 Bruxelles(BE)

(74) Mandataire: de Kemmeter, François et al,
Cabinet Bede 13, avenue Antoine Depage
B-1050 Bruxelles(BE)

(54) **Résinate de metoclopramide et médicament le contenant.**

(57) Dans ce résinate, la Metoclopramide est liée à une résine échangeuse d'ions cationique, de préférence une résine de polystyrène sulfonate d'hydrogène réticulée au divinylbenzène.

Le résinate a de préférence une teneur de 40 % de Metoclopramide et sa granulométrie est avantageusement inférieure à 0,2 mm.

Médicament retard antiémétique.

EP 0 022 117 A2

Croydon Printing Company Ltd.

# Résinate de Metoclopramide

La présente invention est relative au résinate de Metoclopramide.

Dans le présent mémoire, le terme résinate désigne l'association de la Metoclopramide à une résine échangeuse d'ions.

La Metoclopramide ou 4-amino-5-chloro-N-[(2-diéthylamino)éthyl] -2-méthoxybenzamide de formule

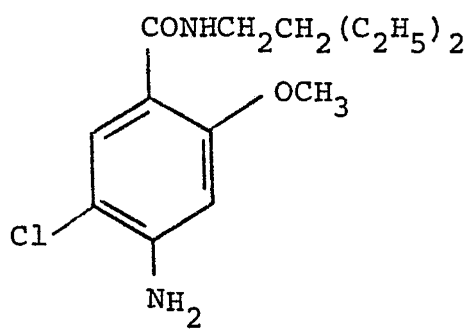

est un médicament antiémétique connu et peut se présenter sous forme de mono- ou de dichlorhydrate monohydraté.

On a constaté que la demi-vie plasmatique de ce médicament a des valeurs très courtes (entre 60 et 90 minutes chez le rat et le chien).

Il a donc paru intéressant de mettre de produit sous forme retard.

Toutefois les formes retard liées à une dissolution lente des comprimés ou d'un enrobage particulier du principe actif (pellets) ne peuvent être utilisées qu'en spécialité car broyées en poudre fine elles perdent complètement leur propriété retard.

Pour éviter ces inconvénients, le demandeur propose de présenter la Metoclopramide sous forme de résinate, l'action retard chimique de cette forme étant liée à une réaction chimique d'échange d'ions dans l'organisme. Ainsi on peut présenter le médicament sous forme d'une poudre contenant une dose de principe actif adaptable à chaque individu, éventuellement en association avec d'autres principes actifs.

Le demandeur a mis au point un résinate de Metoclopramide qui libère le principe actif de façon particulièrement adéquate en donnant régulièrement une concentration constante dans le sang pour une période s'étendant sur environ 12 heures.

On obtient ainsi les avantages liés à une libération progressive et qui consistent en une action constante et régulière du principe actif dans l'organisme sur une période d'environ 12 heures et une diminution des effets secondaires.

Le résinate de Metoclopramide suivant l'invention comprend de préférence la Metoclopramide liée en relation d'échange ionique avec une résine échangeuse de cations.

Une résine cationique appropriée peut être un polystyrène réticulé contenant des groupes d'acide sulfonique ou un polymère d'acide méthacrylique également réticulé ou une autre résine similaire.

L'agent de réticulation est de préférence le divinylbenzène, le degré de réticulation pouvant être compris entre 2 et 9 %, exprimé comme pourcentage en poids d'agent de réticulation dans la résine.

Une résine préférée est la Duolite C-20 constituée par le polystyrène sulfonate d'hydrogène réticulé ou copolymérisé avec le divinylbenzène et comportant des groupements sulfonés, son degré de réticulation est de 8 % et le diamètre des particules, lors de la fabrication du résinate est de 0,3 à 1,2 mm.

Cette granulométrie facilite les filtrations successives nécessaires à cette fabrication.

Le résinate formé est ensuite broyé pour se présenter sous forme de poudre afin de pouvoir effectuer des mélanges homogènes avec d'autres poudres (excipients ou principes actifs) lors des préparations magistrales.

Il n'a pas été possible de choisir une résine de polystyrène sulfonate de sodium car le pourcentage maxima de Metoclopramide que l'on pouvait fixer sur cette résine n'était pas suffisant pour obtenir une libération adéquate du principe actif.

Le choix du pourcentage fixé s'est effectué en fonction de la libération in vitro suivant NF XIII (US National Formulary, 30ième édition, tome XIII), obtenue pour chaque pourcentage fixé.

La prise en résinate de Metoclopramide est ajustée

pour correspondre à une prise de 40 mg de Metoclopramide exprimée en monochlorhydrate monohydraté, à libérer en 12 heures.

Il est apparu que le pourcentage idéal en Metoclopramide fixée est de 40 %.

A noter que 40 % de fixation de Metoclopramide est proche de la saturation de la résine H pour ce produit.

Avec cette teneur en Metoclopramide, la libération progressive du principe actif est optimale lorsque le résinate a une granulométrie inférieure à 0,2 mm obtenue par broyage comme indiqué plus haut.

L'invention est décrite de façon plus détaillée mais non limitative dans l'exemple suivant de préparation d'un résinate de Metoclopramide contenant 40 % de Metoclopramide exprimé en chlorhydrate monohydraté.

EXEMPLE

Pour obtenir 100 kg de résinate de Metoclopramide, on met ensemble les ingrédients suivants :

1) environ l'équivalent de 50 kg de polystyrène sulfonate d'H sec soit 100 kg de Duolite C-20 contenant 50 % d'$H_2O$
2) environ 41 kg de Metoclopramide $HCl.H_2O$
3) environ 120 litres d'eau déminéralisée.

La résine Duolite C-20 est commercialisée par DIA-PROSIM BENELUX, Bruxelles, Belgique.
C'est une résine cationique échangeuse d'ions

consistant en un polystyrène sulfonate d'H réticulé à 8 % par du divinylbenzène . Sa densité apparente est de 0,86 et sa densité réelle de 1,28.

On laisse le mélange décrit ci-dessus sous agitation constante durant 8 heures au minimum puis on laisse reposer une nuit.

La solution surnageante est ensuite décantée et le résinate de Metoclopramide formé est lavé à l'eau déminéralisée jusqu'à ce que les eaux de lavage ne présentent plus de chlorures.

Les opérations de lavage par filtration sont rendues particulièrement aisées grâce à la granulométrie de la résine de départ (comprise entre 0,3 et 1,2 mm).

Le produit est alors séché par distillation sous vide jusqu'à contenir environ 10 % d'$H_2O$ sans dépasser la température de 45°C.

On broie ensuite le produit à l'aide d'un turbomoulin muni d'un tamis circulaire de 0,5 mm et on règle la vitesse de passage de manière à ne pas dépasser une température de 45° - 50°C pour le produit broyé (échauffement dû au frottement lors du broyage).

Le produit ainsi broyé a une granulométrie inférieure à 0,2 mm de diamètre.

Il est à remarquer que le moment du broyage du résinate formé n'a pas d'incidence sur son effet retard. En effet, on obtient les mêmes libérations

in vitro, en broyant la résine au départ et en procédant ensuite à la fixation de la Metoclopramide qu'en procédant comme ci-dessus.

Toutefois, il est préférable d'effectuer la synthèse au départ de la résine non broyée pour éviter tout colmatage des colonnes lors des filtrations successives.

Ce produit présente les caractéristiques suivantes: 40 % Metoclopramide HCl.$H_2O$, 50 % résine, 10 % eau.

1/ Dénomination chimique
Polystyrène sulfonate de Metoclopramide

2/ Caractères
Le résinate de Metoclopramide est une poudre beige, inodore, insoluble dans l'eau, l'éthanol, l'éther et l'acétone.

3/ Identification

1°) Metoclopramide
On met en contact et sous agitation magnétique pendant 30 minutes, 1 g de résinate de Metoclopramide avec 20 ml d'HCl (2N).
La solution est, après centrifugation à ± 4.000 tours/minute pendant 5 minutes, filtrée sur filtre serré et l'insoluble est conservé pour l'identification de la résine.

a) à 5 ml du filtrat on ajoute, en refroidissant, une solution de NaOH (10N) jusqu'à ce que le filtrat soit alcalin. La Metoclopramide donne un précipité blanc, bien soluble dans le chloroforme.

b) 1 ml du filtrat + HCl (0,1N) jusqu'à 500 ml donne le spectre U.V. caractéristique de la

Metoclopramide présentant 2 maxima à $\lambda$ = 308 $\pm$ 1 nm et 272 $\pm$ 1 nm et 2 minima à $\lambda$ = 289 $\pm$ 1 nm et 252 $\pm$ 1 nm.

c) le filtrat neutralisé donne un précipité blanc avec le réactif de Nessler et un précipité blanc jaunâtre avec le réactif de Mayer.

2°) Résine

Mise en évidence de la propriété d'échange cationique de cette dernière.

L'insoluble de 1°) est divisé en $\pm$ 2 parties égales :

- à la première moitié, on ajoute 10 ml d'une solution à 1 % de $CuSO_4 . 5H_2O$ et on agite vigoureusement ⟶ la résine se colore en vert bleu et reste colorée en vert bleu après rinçages successifs à l'$H_2O$.
- à l'autre moitié, on ajoute 10 ml d'une solution à 1 % de $FeCl_3.6H_2O$ et l'on opère de la même manière ⟶ la résine reste colorée en jaune.

3°) On détermine le degré de pureté du produit de façon usuelle pour ce genre de produit.

L'analyse de la Metoclopramide par chromatographie sur couche mince doit faire apparaître sous U.V. à $\lambda$ = 254 nm une seule tache au même $R_F$ que celle provenant de la solution de référence.

L'analyse de la résine doit donner les résultats suivants :

| | |
|---|---|
| Teneur en métaux lourds | : $\leq$ 50 ppm |
| Limite du fer | : $\leq$ 100 ppm |
| Limite du cuivre | : $\leq$ 50 ppm |
| Limite des chlorures | : $\leq$ 100 ppm. |

Le résinate de Metoclopramide peut être présenté par exemple sous forme de comprimés de 100 mg contenant 40 mg de Metoclopramide. Comme posologie moyenne on peut prévoir un comprimé de ce genre par jour.

On peut évidemment aussi envisager d'autres formes galéniques telles que dragées, gélules, tablettes, cachets, poudres, suspensions, gels ou toute autre forme galénique à prendre par voie orale, contenant le résinate associé aux excipients usuels appropriés, éventuellement avec d'autres principes actifs.

## REVENDICATIONS

1. A titre de produit nouveau, le résinate de Metoclopramide.

2. Produit suivant la revendication 1, caractérisé en ce que la Metoclopramide est liée à une résine échangeuse d'ions cationique

3. Produit suivant la revendication 2, caractérisé en ce que la résine échangeuse d'ions cationique est une résine de polystyrène sulfonate d'hydrogène réticulée.

4. Produit suivant la revendication 3, caractérisé en ce que la résine de polystyrènesulfonate d'hydrogène est réticulée au divinylbenzène et a un degré de réticulation d'environ 8.

5. Produit suivant la revendication 4, caractérisé en ce que la résine porte 40 % de Metoclopramide par rapport au poids total du résinate final.

6. Produit suivant la revendication 5, caractérisé en ce que la Metoclopramide mise en oeuvre se présente sous forme de monochlorhydrate monohydraté.

7. Médicament retard à base de résinate de Metoclopramide tel que défini dans l'une quelconque des revendications précédentes.

8. Médicament suivant la revendication 7, dans lequel le résinate de Metoclopramide est en quantité voulue pour correspondre à une prise de 40 mg

de Metoclopramide calculée par rapport au monochlorhydrate monohydraté à libérer en 12 heures.

9. Médicament suivant la revendication 8, présenté sous forme de comprimés, dragées, gélules,tablettes, cachets, poudre, suspension, gel ou toute autre forme galénique à prendre par voie orale, contenant le résinate associé aux excipients usuels appropriés, éventuellement avec d'autres principes actifs.